Europäisches Patentamt

European Patent Office

Office européen des brevets

(19)

(11) Veröffentlichungsnummer : **0 116 349 B1**

# (12) EUROPÄISCHE PATENTSCHRIFT

(45) Veröffentlichungstag der Patentschrift :
16.04.86

(21) Anmeldenummer : 84101089.5

(22) Anmeldetag : 03.02.84

(51) Int. Cl.⁴ : **C 07 C143/02, C 07 C139/14**

(54) **Verfahren zur Isolierung von Paraffinsulfonat aus dem bei der Sulfoxydation von Paraffinen erhaltenen Reaktionsgemisch.**

(30) Priorität : **07.02.83 DE 3304017**

(43) Veröffentlichungstag der Anmeldung :
**22.08.84 Patentblatt 84/34**

(45) Bekanntmachung des Hinweises auf die Patenterteilung : **16.04.86 Patentblatt 86/16**

(84) Benannte Vertragsstaaten :
**DE FR GB IT NL**

(56) Entgegenhaltungen :
**DE-A- 1 568 591**
**GB-A- 347 164**

(73) Patentinhaber : **HOECHST AKTIENGESELLSCHAFT**
**Postfach 80 03 20**
**D-6230 Frankfurt am Main 80 (DE)**

(72) Erfinder : **Pistorius, Rudolf, Dr.**
**Neumühlstrasse 15**
**D-6274 Hünstetten (DE)**

Beschreibung

Die durch Sulfoxidation von n-Paraffinen z. B. nach dem Verfahren des deutschen Patents 910 165 erhältlichen Lösungen von Paraffinsulfonsäuren enthalten außer Schwefeldioxid noch ca. 20 %-ige wäßrige Schwefelsäure und hydrotrop gelöste Paraffine. Um aus solchen Reaktionsgemischen brauchbare Paraffinsulfonate zu isolieren, müssen Schwefeldioxid, Paraffine und Schwefelsäure möglichst weitgehend entfernt werden. Für die Lösung dieser Aufgabe existieren eine Reihe von Lösungsvorschlägen, von denen sich jedoch nur wenige als technisch und wirtschaftlich anwendbar erwiesen haben.

So wird nach dem Verfahren des deutschen Patentes 1910860 das Reaktionsgemisch zunächst durch Erwärmen vom größten Teil des überschüssigen Schwefeldioxids befreit und danach soweit eingedampft, bis sich aus dem Gemisch der überwiegende Teil der nunmehr ca. 60 %-igen Schwefelsäure als Unterphase abscheidet. Die Alkansulfosäure wird dann neutralisiert und die noch das gesamte Paraffin enthaltene Neutralisatlösung bei relativ hohen Temperaturen, d. h. oberhalb ca. 240 °C eingedampft, wobei ein großer Teil des Paraffins azeotrop mitverdampft. Damit der Restparaffingehalt im Endprodukt aber auf einen hinreichend kleinen Wert herabgedrückt werden kann (normalerweise unter 2 Gew.-%), muß zusätzlich mit überhitztem Wasserdampfes nachgestrippt werden.

Bei dieser Verfahrensweise muß nicht zuletzt wegen der hohen Temperaturen in Kauf genommen werden, daß das Produkt relativ stark geschädigt wird, äußerlich an einer intensiven Verfärbung zu erkennen. Infolgedessen muß mit beträchtlichen Wasserstoffperoxidmengen gebleicht werden, um aber zu einem verkaufsfähigen Produkt zu kommen, müssen zusätzlich während des Aufarbeitungsprozesses laufend phosphorhaltige Produkte z. B. Phosphorsäure als Stabilisator zugesetzt werden. Nach diesem Verfahren erhält man zwar ein in der Farbe akzeptables Produkt, dessen, wenn auch kleiner, Phosphatgehalt aber zunehmend als nachteilig empfunden wird. Ebenso wird der größer als 5 % Alkalisulfat (bezogen auf die waschaktive Substanz) relativ hohe Restsalzgehalt und der deutlich wahrnehmbare eigenartige Geruch des Produktes als nachteilig empfunden.

Demgegenüber gelangt man zu phosphatfreien Produkten mit Restsalzgehalten unter 5 % (bezogen auf die waschaktive Substanz) nach dem Verfahren gemäß der deutschen Offenlegungsschrift 1568591, bei dem die Hauptmenge des Paraffins mit niederen Alkoholen, wie auch schon in den deutschen Patentschriften 907052 und 910165 vorgeschlagen, abgetrennt wird und anschließend die saure alkoholische Lösung durch Zugabe konzentrierter Alkalilösungen bei Temperaturen oberhalb 35 °C neutralisiert wird. Das ausgefallene Alkalisulfat wird abfiltriert und danach das restliche Paraffin mit niedrig siedenden Kohlenwasserstoffen extrahiert, um anschließend die Lösungen auf die gewünschte Erdkonzentration einzudicken.

Bei dieser Verfahrensvariante fällt das Natriumsulfat zwar in gut filtrierbarer Form, d.h. relativ grob an, andererseits wird dadurch jedoch verhältnismäßig viel Paraffin und Paraffinsulfonat mitgefällt, die auch durch nachträgliches Waschen mit weiterem Alkohol nicht unter zusammengenommen ca. 2 %, bezogen auf das Gewicht des abgetrennten, getrockneten Alkalisulfates gedrückt werden können. Weiterhin macht der zur Entfernung der restlichen Paraffinmengen erforderliche Einsatz eines niedrigsiedenden Kohlenwasserstoffes das Verfahren sehr kompliziert. Auch die kontinuierliche Eindickung der Alkansulfonatlösungen auf Pasten mit einem Wirkstoffgehalt größer als 50 % waschaktive Substanz läßt sich technisch nur sehr schwer durchführen, weil das Produkt mit zunehmender Eindickung immer viskoser wird, wobei auch bezüglich des Geruchs die auf solche Weise gewonnenen Produkte nicht den gestellten Erfordernissen entsprechen.

Überraschenderweise wurde nun gefunden, daß auf verfahrenstechnisch sehr einfache Weise Produkte erhalten werden können, die sämtlichen Anforderungen genügen, d. h. die arm an Alkalisulfat, phosphatfrei, hell und sehr geruchsarm sind, wenn wie im Folgenden vorgeschlagen, vorgegangen wird.

Gegenstand der Erfindung ist ein Verfahren zur Isolierung von Paraffinsulfonat aus den bei der Sulfoxydation von n-Paraffinen anfallenden Extrakten, aus denen durch Zusatz von Alkohol die Hauptmenge des Paraffins abgetrennt wurde, dadurch gekennzeichnet, daß man die so gewonnenen alkoholischen Lösungen der Paraffinsulfosäuren mit Alkalien in der Weise neutralisiert, daß dabei ein pH-Wert von 7 nicht unterschritten wird und die nach dem Abtrennen des ausgefallenen Alkalisulfats erhaltene Paraffinsulfonatlösung in einem auf mindestens 130 °C vorgeheizten Verdampfer kontinuierlich einengt.

Als Ausgangsmaterial für das Verfahren der vorliegenden Erfindung können die Extrakte beliebiger Sulfoxydations-Verfahren eingesetzt werden. Beispielsweise können die nach den Verfahren der deutschen Patentschriften 735 096, 840 093 oder 903 815 gewinnbaren Extrakte verwendet werden. Es kann ferner auch von Extrakten ausgegangen werden, wie sie bei der Sulfoxydation mit Hilfe von Peroxyden, Ozon oder auch mit $\gamma$-Strahlen, z. B. nach dem Verfahren der deutschen Patentschrift 1 139 116 erhalten werden. Derartige Extrakte weisen oft einen hohen Paraffindisulfonsäuregehalt auf, der aber bei dem Verfahren der vorliegenden Erfindung nicht stört. Bevorzugt nimmt man für das vorliegende Verfahren die bei der Sulfoxydation von n-Paraffinen mit Kettenlängen von etwa 7 bis etwa 18, vorzugsweise 10 bis 18 Kohlenstoffatomen, anfallenden wäßrigen Extrakte. Als Extrakt bezeichnet man die klare Lösung, die man im Anschluß an die Sulfoxydation erhält nach der Abtrennung von ungelöstem Paraffin. Vor der Zugabe des Alkohols kann man diesen Extrakt auch zusätzlich auf 80 bis 105 °C erwärmen. Man erreicht damit eine Vortrennung von ca. 20 bis 33 Gew.-% der im Extrakt insgesamt vorhandenen Menge an Schwefelsäure. Dadurch vermindert sich die für die nachfolgende Neutralisation notwendige Menge an Alkali und es muß am Ende des Verfahrens weniger Wasser abgedampft werden.

Die zum Abscheiden des Paraffins verwendeten niedermolekularen Alkohole wie Methanol, Äthanol und Isopropanol, vorzugsweise Methanol und Äthanol, insbesondere aber Methanol, sollen dem Extrakt in einer Menge von etwa 50 bis 500, vorzugsweise von 100 bis 200 Gewichtsteilen, bezogen auf 100 Gew.-Teile Paraffinsulfonsäuren, zugesetzt werden. Die optimale Menge variiert mit der jeweiligen Zusammensetzung des Extraktes. Als vorteilhaft erweist es sich, dem Extrakt mindestens soviel Alkohol zuzusetzen, daß wenigstens 70 %, vorzugsweise 80-95 % des vorhandenen Paraffins abgeschieden werden. Andererseits kann die für einen gewünschten Paraffinabscheidungsgrad erforderliche Alkoholmenge umso geringer sein, je mehr (verdünnte) Schwefelsäure durch Erwärmen des Reaktionsgemisches auf 80 bis 105 °C und anschließendes Abtrennen der Unterphase entfernt wird, bevor der Alkohol zugesetzt wird.

Ebenfalls in Abhängigkeit von der Zusammensetzung des Reaktionsgemisches und der Menge an Alkohol kann es vorteilhaft sein, den Extrakt bereits vor dem Zusatz des Alkohols teilweise vorzuneutralisieren, d. h. etwa 5 bis 50 %, insbesondere aber 10 bis 30 % der Alkalimenge bereits vor der Alkoholzugabe zuzusetzen, die zu einer vollständigen Neutralisation erforderlich ist. Vor der Alkoholzugabe darf jedoch höchstens soviel Alkali zugesetzt werden, daß nach Zugabe der gewählten Alkoholmenge noch kein Alkalisulfat ausfällt, da letzteres sonst mit dem abgeschiedenen Paraffin eine Suspension bildet, die nur schwer wieder zu trennen ist.

Die Paraffinabscheidung durch Zugabe von Alkohol soll bei 5 bis 55 °C, vorteilhaft bei 15 bis 40 °C, vorzugsweise jedoch bei 18 bis 30 °C vorgenommen werden, wobei dem Gemisch mindestens 5 Minuten, höchstens jedoch 90 Minuten, vorzugsweise 8 bis 20 Minuten Zeit gelassen wird, sich zu trennen.

Das so vom größten Teil des Paraffins befreite Reaktionsgemisch wird unmittelbar darauf bei 10 bis 80 °C, vorzugsweise bei 40 bis 70 °C, so neutralisiert, daß unter intensivem Rühren gleichzeitig Alkali und die alkoholische Paraffinsulfonsäurelösung zusammengegeben werden, wobei die Dosiergeschwindigkeit so eingestellt wird, daß der pH-Wert, gemessen mit einer geeichten pH-Elektrode in dem Gemisch der beiden Flüssigkeiten immer oberhalb 7, insbesondere über 11,0 liegt, normalerweise wird er je nach den Bedingungen der weiteren Aufarbeitung zum Schluß der Neutralisation auf Werte zwischen 10 und 12 eingestellt, damit das Endprodukt einen neutralen bis schwach alkalischen pH-Wert aufweist.

Zur Neutralisation der Paraffinsulfonsäuren können beliebige Alkalien verwendet werden. Es können Natrium- oder Kalium-hydroxid, zweckmäßig in Form konzentrierter wäßriger Lösungen, genommen werden, es ist jedoch auch möglich, Natrium- oder Kaliumcarbonat oder Natrium- oder Kaliumbicarbonat einzusetzen.

Nach Beendigung der Neutralisation wird zur Vervollständigung der Alkalisulfatausfällung auf 10 bis 65 °C, vorteilhaft auf 20 bis 40 °C abgekühlt und die Paraffinsulfonatlösung durch Abdekantieren oder Abschleudern von dem relativ feinteiligen Natriumsulfat befreit.

Das noch mit Paraffinsulfonat und Paraffin behaftete alkoholfeuchte Alkalisulfat kann z. B. vorteilhaft in weiterem Alkohol suspendiert werden und läßt sich jetzt sehr leicht und sauber abfiltrieren und erforderlichenfalls noch waschen, so daß zusammengenommen weniger als 2 %, insbesondere aber weniger als 1 % Paraffinsulfonat und Paraffin, bezogen auf die Alkalisulfatmenge, mit dem Alkalisulfat ausgeschleust werden. Der Suspensions- und Waschalkohol kann unverändert wieder für eine erneute Abscheidung von Paraffin benutzt werden, ohne daß es erforderlich ist, ihn gesondert aufzubereiten.

Die so gewonnene Lösung des Paraffinsulfonats wird nun eingedampft und zwar zunächst soweit, bis der Alkohol vollständig entfernt ist. Die weitere Eindampfung erfolgt in der Weise, daß die Lösung des Paraffinsulfonats langsam in einen Eindampfer gegeben wird, der bereits auf eine Temperatur von mindestens 130° vorgeheizt ist. Die Zugabe der Lösung des Paraffinsulfonats in den Verdampfer wird so geregelt, daß diese Temperatur von 130 °C niemals unterschritten wird. Die optimale Temperatur für diese Aufkonzentrierung liegt zwischen 130 und 200 °C, vorzugsweise zwischen 135 und 180 °C. Diese Temperatur ist abhängig von der Verteilung der Kettelänge in den Alkylgruppen des Paraffinsulfonats. Bei dieser Eindampfung, bei der man eine relativ dünnflüssige Schmelze erhält, geht der größte Teil- in der Regel 70-95 % des Paraffins azeotrop über, das nach in dem Paraffinsulfonat vorhanden ist. Sofern notwendig, können durch Nachstrippen mit wenig überhitztem Wasserdampf bei Sumpftemperaturen von 130 bis 200 °C, insbesondere bei 150 bis 180 °C, schließlich die letzten Spuren von Paraffin entfernt werden. Wird die Aufkonzentrierung der Lösung des Paraffinsulfonats in der beschriebenen Weise durchgeführt, erhält man nach dem Abkühlen der Schmelze ein Produkt mit einem Gehalt von bis zu 95 % an reinem Paraffinsulfonat. Im Gegensatz hierzu liefert das Verfahren nach der DE-PS 15 68 591 Produkte mit einem Gehalt von nur max. 50 %. Dieses hochkonzentrierte Paraffinsulfonat kann dann durch Zugabe von Wasser bei 90 bis 150 °C auf jede gewünschte Konzentration eingestellt werden wobei man dem Wasser zur Beseitigung letzter Geruchsspuren 0,01 bis 0,9, insbesondere 0,2 bis

0,5 % H₂O₂ zumischen kann. Die Vorteile des erfindungsgemäßen Verfahrens sind darin zu sehen, daß das Paraffinsulfonat wesentlich geruchsärmer ist und daß weniger Paraffin und Paraffinsulfonat an dem abgetrennten Alkalisulfat haften, so daß der Verlust an diesen Produkten geringer ist. Außerdem wird hier im Vergleich zu dem Verfahren nach der DE-PS 15 68 591 kein zusätzliches Extraktionsmittel benötigt und man erhält auch das Paraffinsulfonat in einer sehr viel höheren Konzentration.

Beispiel 1

In einer Sulfoxidationsapparatur wurden geradkettige Paraffinkohlenwasserstoffe mit 12 bis 18 Kohlenstoffatomen mit Schwefeldioxid und Sauerstoff in Gegenwart von Wasser und unter Bestrahlung mit UV-Licht bei 30 bis 40 °C umgesetzt. Es wurde ein wäßriger Extrakt erhalten, der durch Erwärmen auf 85 °C unter schwachem Vakuum von Schwefeldioxid befreit wurde. Hiernach bestand der Extrakt aus 36,4 % Wasser, 8,2 % Schwefelsäure, 23,1 % Sulfonsäuren und 32,3 % Paraffinen. 2 433 g dieses entgasten Extraktes wurden soweit abgekühlt, daß sich nach dem Mischen mit 973 g Methanol eine Temperatur von 25 °C einstellte. Innerhalb 15 Minuten scheiden sich als blanke farblose Oberschicht 735 g Paraffin ab. Durch gleichzeitiges Einleiten des vom größten Teil des Paraffins befreiten Extraktes und von 480 g 50 %iger Natronlauge bei 65 °C wurde das Gemisch so neutralisiert, daß der pH-Wert nicht unter einen Wert von 11 sank (gemessen mit einer geeichten Glaselektrode). Zum Schluß der Neutralisation wurde ein pH-Wert von 10,8 eingestellt. Anschließend wurde das Neutralisat auf 30 °C abgekühlt und das Natriumsulfat über einen Druckfilter abfiltriert. Der Natriumsulfatkuchen wurde dreimal mit je 30 g Methanol gewaschen. Das getrocknete Natriumsulfat wog 290 g und enthielt noch 0,6 g Wasser, 2,5 g Alkansulfonat und 0,8 g Paraffin.

Die vom Natriumsulfat befreite Neutralisatlösung (2 972 g) wurde bei Normaldruck bis zu einer Kopftemperatur von 90 °C eingedampft, wobei 984 g Destillat übergingen, auf denen 3 g Paraffin schwammen. Dieses Konzentrat wurde kontinuierlich in einen auf 150 °C-160 °C (Sumpftemperatur) erhitzten Kolben eindosiert, wobei 1 139 g Wasser mit 41 g Paraffin übergingen. Durch Nachstrippen mit 50 g auf 180 °C überhitzten Wasserdampf konnte aus der auf 160 °C gehaltenen Schmelze nochmals 3 g Paraffin ausgetrieben werden.

Durch Zugabe von 375 g Wasser und 3 g Wasserstoffperoxid bei 90 bis 100 °C erhält man 1 000 g einer 60 %igen Paraffinsulfonatpaste, die noch 0,97 % Natriumsulfat und 0,8 % Paraffin enthält und die nahezu farb- und geruchslos ist.

Beispiel 2

Verwendet wurde derselbe entgaste Extrakt wie im Beispiel 1, von dem jedoch 2 433 g vor der Methanolzugabe mit 100 g 50 %iger Natronlauge vorneutralisiert wurden. Unter identischen Bedingungen wie im Beispiel 1 scheiden sich jedoch 740 g Paraffin als Oberphase ab. Die übrige Aufarbeitung verlief analog zu Beispiel 1, d. h. zur vollständigen Neutralisation wurden nochmals 380 g 50 %ige Natronlauge benötigt und beim Eindampfen zur Schmelze gingen mit 1 140 g Wasser 39 g Paraffin über — das Endprodukt (998 g) enthält noch 0,5 % Paraffin und 0,9 % Natriumsulfat und ist praktisch farb- und geruchslos.

Beispiel 3

Vom gleichen entgasten Extrakt wie in Beispiel 1 beschrieben, wurden 2 433 g auf 90 °C erwärmt und nach 6 minütiger Absetzzeit als untere Phase 285,6 g 20,5 %-ige Schwefelsäure, die noch 0,6 % Alkansulfonsäuren enthält, abgeschieden. Unter den gleichen Bedingungen wie in Beispiel 1 wurden 860 g Methanol zugesetzt, worauf sich nach 15 Minuten 741 g Paraffin als obere Phase abtrennen ließen. Hierauf wurde ebenfalls unter den gleichen Bedingungen wie in Beispiel 1 mit 365 g 50 %-iger Natronlauge neutralisiert. Nach dem Abkühlen des Neutralisates auf 30 °C wurde über eine Drucknutsche das ausgefallene Natriumsulfat (187 g) abgetrennt, das nach dem 3-maligen Waschen mit je 20 ml Methanol und nach 3-stündigem Trocknen bei 85 °C noch 0,5 g Wasser, 2,7 g Alkansulfonat und 0,7 Paraffin enthält. Die vom Natriumsulfat befreite Neutralisatlösung (2 330 g) wurde bei Normaldruck bis zu einer Kopftemperatur von 90 °C eingedampft, wobei 862 g Destillat übergingen, auf dem 5 g Paraffin schwammen. Dieses Konzentrat wurde kontinuierlich in einen auf 150-160 °C (Sumpftemperatur) erhitzten Kolben eindosiert, wobei 765 g Wasser mit 34 g Paraffin übergingen. Durch Nachstrippen mit 300 g auf 180 °C überhitzten Wasserdampf konnten aus der auf 160 °C gehaltenen Schmelze nochmals 10 g Paraffin ausgetrieben werden.

Durch Zugabe von 410 g Wasser und 3 g Wasserstoffperoxid bei 90 bis 100 °C erhält man 1 000 g einer 60 %-igen Paraffinsulfonatpaste, die noch 0,6 % Natriumsulfat und 1,6 % Paraffin enthält und die nahezu farb- und geruchslos ist.

**Patentansprüche**

1. Verfahren zur Isolierung von Paraffinsulfonat aus den bei der Sulfoxydation von n-Paraffinen anfallenden Extrakten, aus denen durch Zusatz von Alkohol die Hauptmenge des Paraffins abgetrennt wurde, dadurch gekennzeichnet, daß man die so gewonnenen alkoholischen Lösungen der Paraffinsulfonsäuren bei einer Temperatur von 10 bs 80 °C mit Alkalien in der Weise neutralisiert, daß dabei ein pH-Wert von 7 nicht unterschritten wird und die nach dem Abtrennen des ausgefallenen Alkalisulfats erhaltene Paraffinsulfonatlösung in einem auf mindestens 130 °C vorgeheizten Verdampfer kontinuierlich einengt.

2. Verfahren nach Anspruch 1, dadurch gekennzeichnet, daß man dem Extrakt bereits vor der Zugabe des Alkohols 5 bis 50 % der zur Neutralisation erforderlichen Gesamtmenge an Alkali zugibt.

## Claims

1. A process for isolating paraffinsulfonate from the extracts which are obtained on sulfoxidating n-paraffins and from which the bulk of the paraffin has been separated by adding alcohol, which comprises neutralizing the alcoholic paraffinsulfonic acid solutions thus obtained with alkali metal hydroxides at a temperature from 10 to 80 °C in such a way as not to drop below pH 7, and continuously concentrating in an evaporator preheated at at least 130 °C, the paraffinsulfonate solution obtained on separating off the precipitated alkali metal sulfate.

2. The process as claimed in claim 1, wherein 5 to 50 % of the total amount of alkali necessary for the neutralization is added to the extract before the alcohol is added.

## Revendications

1. Procédé pour isoler un paraffinesulfonate des extraits fournis par la sulfoxydation de n-paraffines, desquels on a séparé la majeure partie de la paraffine par addition d'un alcool, procédé caractérisé en ce que les solutions alcooliques des acides paraffine-sulfoniques qui ont été ainsi obtenues sont neutralisées par des composés alcalins à une température de 10 à 80 °C, cela de telle façon que le pH ne tombe pas au-dessous de 7, et la solution de paraffine-sulfonate obtenue après séparation du sulfate de métal alcalin qui a précipité est concentrée continuellement dans un évaporateur préalablement chauffé à au moins 130 °C.

2. Procédé selon la revendication 1 caractérisé en ce qu'on ajoute à l'extrait, déjà avant d'y ajouter l'alcool, de 5 à 50 % de la quantité totale de composé alcalin nécessaire pour la neutralisation.